# FASCICULE DE BREVET EUROPEEN

(11) **EP 4 349 318 B1**
(45) Date de publication et mention de la délivrance du brevet: **16.07.2025**
(21) Numéro de dépôt: 23188519.5
(22) Date de dépôt: 28.07.2023
(51) Int. Cl.: A61K 8/02, A61K 8/11, A61K 8/25, A61K 8/34, A61K 8/46, A61K 8/73, A61Q 5/02, A61Q 19/10

(54) **COMPOSITION COSMÉTIQUE SOUS FORMES DE BILLES SÈCHES NETTOYANTES**
KOSMETISCHE ZUSAMMENSETZUNG IN FORM VON TROCKENEN REINIGUNGSKUGELN
COSMETIC COMPOSITION IN THE FORM OF CLEANSING DRY BEADS

(30) Priorité: 05.10.2022 FR 2210207
(43) Date de publication de la demande: 10.04.2024
(73) Titulaire: Technature, 29460 Dirinon (FR)
(72) Inventeur: VALLEE, Lucas, 29800 LANDERNEAU (FR); COZ, Anne-Louise, 29450 SIZUN (FR)
(74) Mandataire: Bringer IP

(56) Documents cités:
- US-A1- 2006 228 319

## Description

### Domaine technique

L'invention porte sur une composition cosmétique se présentant sous forme de billes expansées sèches obtenues par lyophilisation, dont la mise en œuvre par réhydratation et action mécanique permet l'obtention d'une solution nettoyante. Celle-ci répond à l'intérêt porté aux produits anhydres par l'industrie cosmétique de par la diminution de leur impact environnemental en raison de la diminution des conditionnements et de la quantité d'eau à transporter, ainsi que la réduction ou l'absence de produits conservateurs.

### Technique antérieure

La fabrication de produits se présentant sous forme de billes sèche est largement décrite dans la littérature sous forme expansée, leur réalisation faisant principalement appel à deux familles de procédés, par extrusion ou par lyophilisation.

Les procédés par extrusion consistent à compresser un matériau, soit à chaud, soit à froid, et pouvant se combiner à une réaction chimique ou enzymatique. Ces procédés offrent un grand nombre d'applications possibles tant en termes de formes que de galéniques de produits finis. Les extrudeuses les plus abouties permettent de contrôler la pression et la température en tout point du procédé. Le contrôle de la pression ainsi que du malaxage des ingrédients se fait par le biais d'une ou plusieurs vis sans fin. Ces systèmes permettent d'assurer un procédé de fabrication en continu et peu onéreux, et permettent aussi de modifier la physicochimie d'un produit fini.

Cette technique est notamment utilisée dans le domaine agroalimentaire, pour produire des dérivés de céréales tels que des snacks apéritifs ou des tartines croustillantes de type « cracotte » (marque déposée), leur structure expansée étant obtenue notamment en raison de leurs teneurs élevées en polysaccharides. Les ingrédients sont mélangés à une température de 60 à 80°C afin d'obtenir une pâte épaisse, la température étant ensuite augmentée jusqu'à 120 à 140°C tout en augmentant la pression pour empêcher l'eau présente dans le produit de bouillir, ce qui permet l'expansion du produit lors de la sortie du produit, l'abaissement rapide de la pression provoquant une libération de l'eau sous forme gazeuse. (ROUSTEL, S. Cuisson extrusion des aliments. Techniques de l'ingénieur, septembre 2000, F3120V1, 1-8).

Les procédés par lyophilisation consistent à extraire l'eau contenue dans des produits en provoquant la sublimation de l'eau qu'ils contiennent en les plaçant, préalablement congelés, dans une enceinte sous vide. Dans un premier temps, une solution aqueuse comportant généralement des polysaccharides est coulée dans des moules à la forme voulue, ou coulée en goutte à goutte et précipitée dans un liquide, est ensuite congelée à une température de l'ordre de -25°C à -40°C, puis est placée sous vide en étant modérément réchauffée, afin de rester sous le point triple de l'eau, permettant ainsi la sublimation de l'eau sans altération du réseau préalablement formé par les polysaccharides en solution. (MARIN, M. et RENE, F. Lyophilisation. Techniques de l'ingénieur, mars 2000, F3240V1, 1-9).

Nonobstant la forme spécifique de l'invention, les produits de ce type sont proches, en termes de propriétés physico-chimiques, de produits se présentant sous forme de films hydrosolubles. De nombreux travaux relatifs à la formulation de films hydrosolubles sont décrits, ceux-ci sont notamment utilisés en cosmétique pour leurs propriétés d'adhésion, de résistance à l'étirement, leur effet barrière à l'oxydation, leurs propriétés filmogènes ainsi que pour leur effet tenseur. Cependant, dans ce domaine, très peu de travaux sont rapportés concernant les films hydrosolubles expansés.

Les propriétés de ces films sont évaluées par deux types de mesures :
1. Le temps de restitution/dissolution en milieu aqueux. Ce facteur permet d'évaluer la résistance du film au degré d'hygrométrie ambiant. L'application cosmétique nécessitant une dissolution rapide et simple afin de permettre au consommateur une utilisation aisée du produit fini, il est donc nécessaire de trouver un équilibre entre vitesse de restitution et facilité d'utilisation (SAINI, S et al. Optimization of formulation of fast dissolving films made of pullulan polymer. International Journal of Pharmaceutical Sciences Review and Research, Août 2011, 9(1), 127-131).
2. La résistance du film à l'étirement/torsion. Ce facteur permet d'améliorer la préhensibilité du film par le consommateur. Il est caractérisé par la résistance à l'étirement (σ) et le module d'élongation (ε) ainsi que la force de rupture (JOST, V. et STRAMM, C. Influence of plasticizers on the mechanical and barrier properties of cast biopolymer films. Journal of Applied Polymer Science, 2015, 133(2)). Et (KARKI, S. et al. Thin films as an emerging platform for drug delivery. Asian Journal of Pharmaceutical Sciences, Octobre 2016, 11(5), 559-574).

Par ailleurs, il est possible de jouer sur l'épaisseur du film et sa composition. La formulation de films hydrosolubles est bien connue de l'homme de l'art dans le domaine médical, où cette galénique est utilisée comme moyen de libération de molécules actives. Elle repose sur les relations synergiques entre des molécules filmogènes telles que des polysaccharides ou des protéines et des molécules plastifiantes tels que certains oligosaccharides ou glycols (SANYANG, M.L. et al. Effect of glycerol and sorbitol plasticizers on physical and thermal properties of sugar palm starch based films. Recent Advances in Environment, Ecosystems and Development, avril 2015, 157-162). Les polysaccharides forment un réseau tridimensionnel structuré, lequel permet la formation d'un film cohésif lorsque le solvant s'évapore, alors que les plastifiants permettent de conférer à ce film les caractéristiques mécaniques souhaitées pour celui-ci, notamment sa souplesse et sa texture.

Parmi les composés filmogènes, le pullulan est l'un des polysaccharides les plus utilisés pour la formulation de films hydrosolubles, en raison de sa solubilité élevée et de sa plasticité qui facilite la formulation de produits en limitant l'adjonction de substances plastifiantes. C'est un homopolysaccharide de glucose branché en α(1-6) linked maltotriose, obtenu par fermentation d'amidon de blé par le champignon Aureobasidium pullulans. (LEATHERS, T. Biotechnological production and applications of pullulan. Applied Microbiology and Biotechnology, Juin 2003, 62:468-473.).

Cependant de nombreux autres polysaccharides sont connus pour être utilisés dans la formulation de film hydrosolubles, permettant d'obtenir différentes caractéristiques, notamment les amidons, les alginates, les carraghénanes, et les dérivés de cellulose tels que l'hydroxymethylcellulose. L'article (FERREIRA, A. et al. Polysaccharide-based membranes in food packaging applications. Membranes, Avril 2016, 6(2), 1-17.) détaille leurs principales caractéristiques.

Les composés utilisés comme plastifiants présentent un encombrement stérique plus faible, ce qui leur permet de venir se loger au sein du réseau formé par les polysaccharides, y modifiant la résistance, l'élasticité, la flexibilité, l'aspect et la vitesse de dissolution du film. Parmi ceux-ci nous pouvons dénombrer les glycols, tels que la glycerine et le polyéthylène glycol, les oligosaccharides tels que le sorbitol, le mannitol, le xylitol (SOTHORNVIT, R. et KROCHTA, J. Plasticizers in edible films and coatings. Innovations in Food Packaging, 2005, 403-433.) et (La transition vitreuse. University Mississipi. 1998). Par ailleurs, les colloïdes tels que les silices et les argiles peuvent être utilisés de façon limitée, car ne contribuant pas à la formation d'un réseau gélifié, ils ont tendance à fragiliser le film déshydraté, et diminuent par conséquent la température de transition vitreuse.

Concernant la réalisation de billes, à la différence de films hydrosolubles pour lesquels l'incorporation de particules colloïdales impacte négativement la stabilité du film, l'incorporation de colloïdes permet d'alléger le réseau matriciel généré par les polysaccharides. En effet, la nature d'une dispersion colloïdale (MONGONDRY, P. Structure et comportement rhéologique des suspensions aqueuses de Laponite en présence de plusieurs additifs. Analyse de données, Statistiques et Probabilités, Thèse de chimie et physico-chimie des polymères de l'Université du Maine, Le Mans, juin 2003, 6-20.) implique des propriétés rhéologiques particulières telles qu'une importante rhéofluidification ainsi qu'une thixotropie permettant, par exemple, une meilleure pulvérisation de suspensions colloïdales à haute viscosité.

Concernant la réalisation de billes expansées dans le domaine cosmétique, leur spécificité réside essentiellement dans la mise en œuvre mécanique permettant de donner cette forme physique au produit. Peu de publications rapportent de telles réalisations, parmi lesquelles on peut cependant citer :
1. La demande de brevet CN109350555 A (JIANGSU JLAND BIOTECH CO) 19 février 2019, revendication 7, paragraphes [0032] à [0043] expose le procédé de fabrication de billes de collagène, recourant à une technique de lyophilisation, mais diffère notablement par la composition du produit de la présente invention, recourant notamment à des techniques de fermentation, et par ailleurs ne décrivant pas précisément la technique de formation des billes ni les conditions de la lyophilisation.
2. La demande de brevet CN112891230 A (GUANGZHOU RIDGEPOLE BIOLOGI-CAL TECH CO) 4 juin 2021, revendications 1 à 9, paragraphes [0005] à [0083], expose la formulation et le procédé de fabrication de billes à usage cosmétique obtenues par un procédé de lyophilisation, et comportant notamment un polyol tel que le sorbitol et un polysaccharide, dont le pullulan. La composition est mise en œuvre par la réalisation d'une solution, laquelle est congelée à -45°C dans un moule hémisphérique, et lyophilisée sous un vide de 10 à 30 Pa. Cette demande de brevet diffère cependant de la présente invention en ce que son objet ne concerne pas la composition de produits permettant de réaliser des billes dont la mise en œuvre permet d'obtenir un produit nettoyant, et que la composition ne mentionne pas l'utilisation d'un agent plastifiant tel que la glycérine ; par ailleurs, cette demande ne rapporte pas non plus l'utilisation de colloïdes dans les formulations exposées.
3. Le brevet KR102187653 B1 (BEAUTIFUL KOREA CO) 27 août 2020, revendications 1, 18 et 19, paragraphes [0007] à [0073], expose une composition cosmétique obtenue par lyophilisation pouvant être sous forme sphérique. Cependant, ce brevet diffère de la présente invention en ce qu'il concerne des produits à base de mannitol et d'acide hyaluronique destinés à l'obtention de produits hydratant et antirides.

La publication de la demande de brevet US 2006/0228319 A1 divulgue dans l'exemple 4 des compositions nettoyantes sous la forme d'un film sec constitué de mélanges d'amidon modifié au pullulan contenant du pullulan, de la glycérine, de l'amidon de maïs et du lauryl éther sulfate de sodium.

A la connaissance de la société demanderesse, il n'existe pas de publication décrivant la formulation et la fabrication de billes expansées permettant l'obtention d'une solution nettoyante après réhydratation.

### Exposé de l'invention

La présente invention ayant pour objet la réalisation de billes nettoyantes sèches obtenues par lyophilisation, elle consistera dans sa première étape en la réalisation d'une solution aqueuse destinée à être lyophilisée, dont d'une part la composition permettra d'obtenir les caractéristiques physico-chimiques recherchées pour des billes nettoyantes sèches, ré-hydratables et pouvant être mises en œuvre par action mécanique et d'autre part la viscosité sera compatible avec la formation de gouttes selon le procédé de fabrication.

La seconde étape du procédé de lyophilisation consistera en la surgélation de cette solution, dans des conditions permettant l'obtention de billes. Préférentiellement, on utilise un système consistant à délivrer en goutte à goutte la solution dans de l'azote liquide à une température de - 196°C, ce qui permet de figer celles-ci par une congélation ultra rapide. Ainsi, ce système consiste à envoyer la solution dans une rampe comportant plusieurs buses dont la géométrie, notamment leur forme et la taille de leurs orifices de sortie, est adaptée parallèlement à la rhéologie de la solution et à son débit à travers ces buses, afin de permettre l'obtention de gouttes du diamètre recherché, qui sera compris entre 1 et 8 millimètres, selon les caractéristiques souhaitées pour le produit final. Préférentiellement, une viscosité comprise entre 1000 et 30000 mPa.s sera recherchée, et des buses d'un diamètre compris entre 1 et 6 mm pourvues d'une sortie évasée seront utilisées, une taille de billes de l'ordre de 5 à 8 mm correspondant à l'ordre de grandeur de la taille maximale pouvant être obtenue par ce procédé.

Secondairement, un autre mode de réalisation de l'invention consiste à couler la solution dans des moules sphériques ou hémisphériques, ces moules étant ensuite congelés par refroidissement, ce qui permet l'obtention de billes d'un diamètre supérieur, compris entre 5 mm et 25 mm.

Enfin, le procédé de fabrication comporte une troisième étape, celle de lyophilisation, pendant laquelle les billes de produit précédemment obtenues, maintenues à une très basse température sont introduites dans le lyophilisateur, puis exposées au vide et légèrement réchauffées pendant la durée nécessaire au processus de lyophilisation. Préférentiellement, on utilisera des conditions de vide comprises entre 2 et 25 Pa, une température de chargement comprise entre -50 et -150 °C, une température de réchauffage comprise entre 25°C et 50°C, et une durée de lyophilisation comprise entre 2 heures et 16 heures, avec un condensateur à une température comprise entre -30 et -80°C, les conditions exactes dépendant de la quantité de billes à traiter et des caractéristiques dimensionnelles et fonctionnelles du lyophilisateur.

Les billes expansées sèches obtenues après cette étape de lyophilisation pourront être utilisées en les plaçant au creux de la main, puis en versant sur celles-ci 1 à 5 ml d'eau, et ensuite en exerçant une friction des deux mains l'une contre l'autre, provoquant ainsi le délitement des billes et l'obtention de l'action nettoyante recherchée.

L'objet de la présente invention étant la conception de billes nettoyantes sèches, la composition de la solution comporte un ou plusieurs produits tensioactifs, à une teneur (massique) globale comprise entre 0.1 et 10%. Ceux-ci seront préférentiellement choisis parmi les composés se présentant à l'état pur et à température ambiante sous forme de poudre, celle-ci étant la forme physique la plus aisément compatible avec la réalisation finale de billes sèches et solides susceptibles d'être réhydratées.

Parmi ces tensioactifs, on utilisera notamment un mélange de tensioactifs non-ioniques et de tensioactifs anioniques, lesquels seront préférentiellement choisis parmi les dérivés de sodium cocoyl notamment des tensioactifs anioniques moussants doux dérivés de sodium cocoyl, tels que les sodium methyl cocoyl taurate, sodium cocoyl isethionate, sodium cocoyl glutamate ou du disodium lauryl succinate, pouvant être utilisés en association avec des agents tensioactifs non-ioniques pour pallier au côté irritant des anioniques.

Dans une variante de l'invention, pour l'obtention de shampoing, on pourra également utiliser des tensioactifs cationiques, préférentiellement choisis parmi les ammoniums quaternaires tels que le polyquaternium-7.

De même que dans l'état de l'art précité, on utilisera un agent filmogène constitué de polysaccharides permettant l'obtention après lyophilisation d'un réseau matriciel tridimensionnel fin et hydrosoluble ; ses constituants seront choisis parmi le pullulan, les alginates, les carraghénanes, les gommes de xanthane ou de guar ou de cellulose, seuls ou en mélange, leur teneur (massique) globale étant comprise entre 0.5 et 10%.

Préférentiellement mais non exclusivement, on utilisera le pullulan a une teneur comprise entre 0.5 et 5%, en raison de l'aptitude particulière de ce composé à permettre l'obtention des caractéristiques physico-chimiques les plus proches de celles recherchées, légèrement collant sur la peau et présentant un bon compromis entre souplesse et caractère cassant lors de l'application mécanique du produit final sur la peau ou les cheveux. Concernant les autres polysaccharides précités, utilisables conjointement au pullulan, leur teneur globale sera comprise entre 0.1% et 5% ; préférentiellement, les gommes de xanthane ou de guar ou de cellulose seront utilisées.

De même que dans l'état de l'art, dans le but d'améliorer les caractéristiques mécaniques des billes, notamment leur souplesse et leur texture, on utilise un agent plastifiant choisi parmi les glycols et les polyols, tels que la glycérine, le polyéthylène glycol, le sorbitol, le mannitol, le xylitol, ou des oligosaccharides tels que le saccharose, à une teneur (massique) globale comprise entre 0.5 et 10%.

Préférentiellement, on utilisera un mélange de glycérine à une teneur comprise entre 0.5 et 3%, et d'érythritol à une teneur comprise entre 0.5 et 3%, en raison de l'incidence particulièrement favorable de ce polyol sur la douceur de la texture du produit final.

Comme attendu au vu de l'état de l'art, les caractéristiques rhéologiques du produit peuvent être améliorées par l'adjonction d'un agent rhéologique colloïdal, permettant ainsi d'améliorer son aptitude à former des gouttes de tailles importantes. Cependant, la société demanderesse a constaté, de façon inattendue, que l'adjonction d'un mélange spécifique constitué d'une part de cellulose microcristalline ou de colloïdes tels que les silices ou les argiles, et d'autre part d'une poudre d'amidon, permet d'améliorer la capacité à la réhydratation du produit final, alors que l'utilisation seule d'un gélifiant constitué de polysaccharides ne peut améliorer la rhéologie qu'au prix d'une dégradation de ces caractéristiques de réhydratation. Cet agent rhéologique entrera dans la composition à une teneur globale comprise entre 0.1 et 10%.

Préférentiellement, l'agent rhéologique choisi sera composé d'un mélange d'amidon à une teneur comprise entre 2.5 et 7%, associé à de la cellulose microcristalline ou de la bentone, à une teneur comprise entre 0.25 et 1.5%.

Enfin, la composition comportera les ingrédients ou actifs dont l'utilisation est connue de l'homme de l'art pour des produits cosmétiques, tels que les colorants, parfums, actifs et conservateurs.

### Description des modes de réalisation

Ainsi, ces travaux ont permis d'aboutir à la réalisation de billes sèches nettoyantes, d'un diamètre compris entre 1 et 8 mm ou entre 6 et 25 mm selon la variante de procédé de fabrication utilisée, pouvant être aisément mise en œuvre en plaçant celles-ci au creux de la main et se délitant spontanément ou par action mécanique d'un doigt en présence de quelques millilitres d'eau, et permettant par suite un nettoyage efficace des mains, de toute autre partie du corps, dont la chevelure.

On donne maintenant à titre d'exemples des formules et des modes de fabrication.

### Exemples

### Exemple 1.

### Etape 1 - Réalisation de la solution à lyophiliser

Mode opératoire : (1) Introduire l'eau, le colorant, l'extrait de riz, le pullulan, l'érythritol l'alpha-glucane oligosaccharide et l'amidon de riz dans un mélangeur et agiter jusqu'à homogénéisation. (2) Prédisperser les gommes de xanthane et de cellulose, la cellulose cristalline dans la glycérine puis ajouter à la phase précédante en mélangeant énergiquement jusqu'à homogénéisation jusqu'à obtention d'un gel lisse et homogène. Ajouter ensuite le disodium lauryl sulfosuccinate, le sodium cocoyl isethionate sous agitation lente, puis le parfum. Enfin, ajuster le pH entre 4.5 et 6.5 avec la solution d'acide citrique. La viscosité de la solution ainsi réalisée se situe dans une fourchette comprise entre1000 et 6000 mPa.s.

**[Table 1]**

| Produits | % (massiques) |
|---|---|
| EAU | 82.784 |
| COLORANT | 0.015 |
| EXTRAIT DE RIZ | 0.100 |
| PULLULAN | 0.900 |
| ERYTHRITOL | 1.500 |
| ALPHA-GLUCANE OLIGOSACCHARIDE | 0.500 |
| AMIDON DE RIZ | 5.000 |
| GOMME DE CELLULOSE | 0.290 |
| GOMME DE XANTHANE | 0.200 |
| CELLULOSE MICROCRISTALLINE | 0.510 |
| GLYCERINE | 1.500 |
| DISODIUM LAURYL SULFOSUCCINATE | 3.700 |
| SODIUM COCOYL ISETHIONATE | 2.600 |
| PARFUM | 0.400 |
| ACIDE CITRIQUE 5% | 0.001 |
| | 100.00 |

### Etape 2 - Lyophilisation

Introduire la solution à un débit ajusté afin de permettre un écoulement en goutte à goutte dans un bain d'azote liquide à - 196°C, en utilisant une pompe péristaltique et une rampe de buses présentant des orifices de sortie évasés, ce qui assure la formation quasi-instantanée de billes congelées d'une taille d'environ 5 mm.

Récupérer ensuite les billes congelées, et les placer dans un lyophilisateur avec une température de chargement de -120°C. Appliquer alors un vide de 10 Pa, puis une température de 30°C, le condenseur étant maintenu à une température de -65°C, ces conditions étant maintenues pendant une durée de 12 heures, à l'issue de laquelle il est possible de récupérer les billes lyophilisées.

### Description de la mise en œuvre pour utilisation.

Placer une bille au creux de la main, et ajouter sur celle-ci quelques millilitres d'eau. Frictionner la bille entre les deux mains l'une contre l'autre jusqu'à ce que la bille se délite, ce qui permet d'obtenir l'action nettoyante recherchée. Rincer après application.

### Exemple 2.

### Etape 1 - Réalisation de la solution à lyophiliser

Mode opératoire : (1) Introduire l'eau, le colorant, l'extrait de lotus, le pullulan, le xylitol et un extrait de levure *Saccharomyses cerverisae* et l'amidon de pomme de terre dans un mélangeur et agiter jusqu'à homogénéisation. (2) Prédisperser la gomme de cellulose, l'extrait d'algues brunes, et la cellulose microcristalline dans le butylène glycol et ajouter à la phase précédente en mélangeant énergiquement jusqu'à jusqu'à obtention d'un gel lisse et homogène. Ajouter ensuite le disodium lauryl sulfosuccinate, le sodium cocoyl isethionate sous agitation lente, puis le parfum. Enfin, ajuster le pH entre 4.5 et 6.5 avec la solution d'acide citrique. La viscosité de la solution ainsi réalisée se situe dans une fourchette comprise entre 1000 et 6000 mPa.s.

**[Table 2]**

| Produits | % (massiques) |
|---|---|
| EAU | 83.539 |
| COLORANT | 0.010 |
| EXTRAIT DE LOTUS | 0.100 |
| PULLULAN | 0.800 |
| XYLITOL | 1.700 |
| EXTRAIT DE LEVURE SACCHAROMISES CEREVISAE | 0.100 |
| AMIDON DE POMME DE TERRE | 4.000 |
| GOMME DE CELLULOSE | 0.100 |
| EXTRAIT D'ALGUES BRUNES | 0.250 |
| CELLULOSE MICROCRISTALLINE | 0.450 |
| BUTYLENE GLYCOL | 1.500 |
| DISODIUM LAURYL SULFOSUCCINATE | 4.000 |
| SODIUM COCOYL ISETHIONATE | 3.100 |
| PARFUM | 0.350 |
| ACIDE LACTIQUE 50% | 0.001 |
| | 100.00 |

### Etape 2 - Lyophilisation

Introduire la solution à un débit ajusté afin de permettre un écoulement en goutte à goutte dans un bain d'azote liquide à - 196°C, en utilisant une pompe péristaltique et une rampe de buses présentant des orifices de sortie évasés, ce qui assure la formation quasi-instantanée de billes congelées d'une taille d'environ 5 mm.

Récupérer ensuite les billes congelées, et les placer dans un lyophilisateur avec une température de chargement de -120°C. Appliquer alors un vide de 10 Pa, puis une température de 30°C, le condenseur étant maintenu à une température de -65°C, ces conditions étant maintenues pendant une durée de 12 heures, à l'issue de laquelle il est possible de récupérer les billes lyophilisées.

### Description de la mise en œuvre pour utilisation.

Placer une bille au creux de la main, et ajouter sur celle-ci quelques millilitres d'eau. Frictionner la bille entre les deux mains l'une contre l'autre jusqu'à ce que la bille se délite, ce qui permet d'obtenir l'action nettoyante recherchée. Rincer après application.

### Exemple 3.

### Etape 1 - Réalisation de la solution à lyophiliser

Mode opératoire : (1) Introduire l'eau, le colorant, l'extrait de thé vert, le pullulan, le mannitol, l'extrait de graine de lin et l'amidon de riz dans un mélangeur et agiter jusqu'à homogénéisation. (2) Prédisperser la gomme de guar, l'extrait d'algues rouges et la bentonite hydrophile dans le butylène glycol puis ajouter à la phase précédente en mélangeant énergiquement jusqu'à homogénéisation la butylène glycol dans un second mélangeur, puis ajouter progressivement jusqu'à obtention d'un gel lisse et homogène. Ajouter ensuite le disodium lauryl sulfosuccinate, le sodium methyl oleyl taurate, le copolymère de 2-propénamide sous agitation lente un à un, puis le parfum. Enfin, ajuster le pH entre 4.5 et 6.5 avec la solution d'acide citrique. La viscosité de la solution ainsi réalisée se situe dans une fourchette comprise entre 1000 et 6000 mPa.s.

**[Table 3]**

| Produits | % (massiques) |
|---|---|
| EAU | 81.883 |
| COLORANT | 0.017 |
| EXTRAIT DE THE VERT | 0.100 |
| PULLULAN | 1.100 |
| MANNITOL | 1.200 |
| EXTRAIT DE GRAINE DE LIN | 0.500 |
| AMIDON DE RIZ | 3.000 |
| GOMME DE GUAR | 0.200 |
| EXTRAIT D'ALGUES ROUGES | 0.500 |
| BENTONITE HYDROPHILE | 1.000 |
| BUTYLENE GLYCOL | 1.500 |
| DISODIUM LAURYL SULFOSUCCINATE | 3.500 |
| SODIUM METHYL OLEYL TAURATE | 2.200 |
| COPOLYMÈRE DE 2-PROPÉNAMIDE ET DE CHLORURE DE N,N-DIMÉTHYL-N-2-PROPÉNYL-2-PROPÈNE-1-AMINIUM | 1.500 |
| PARFUM | 0.500 |
| ACIDE CITRIQUE 35% | 0.300 |
| | 100.00 |

### Etape 2 - Lyophilisation

Introduire la solution à un débit ajusté afin de permettre un écoulement en goutte à goutte dans un bain d'azote liquide à - 196°C, en utilisant une pompe péristaltique et une rampe de buses présentant des orifices de sortie évasés, ce qui assure la formation quasi-instantanée de billes congelées d'une taille d'environ 5 mm.

Récupérer ensuite les billes congelées, et les placer dans un lyophilisateur avec une température de chargement de -120°C. Appliquer alors un vide de 10 Pa, puis une température de 30°C, le condenseur étant maintenu à une température de -65°C, ces conditions étant maintenues pendant une durée de 12 heures, à l'issue de laquelle il est possible de récupérer les billes lyophilisées.

### Description de la mise en œuvre pour utilisation.

Placer une bille au creux de la main, et ajouter sur celle-ci quelques millilitres d'eau. Frictionner la bille entre les deux mains l'une contre l'autre jusqu'à ce que la bille se délite, puis appliquer sur les cheveux en frottant, ce qui permet d'obtenir l'action nettoyante recherchée pour un shampoing. Rincer après application.

### Exemple 4.

### Etape 1 - Réalisation de la solution à lyophiliser

Mode opératoire : (1) Introduire l'eau, l'extrait de riz, le pullulan, le sorbitol, l'alpha-glucane oligosaccharide et l'amidon de maïs et la silice colloïdale dans un mélangeur et agiter jusqu'à homogénéisation. (2) Introduire la glycérine dans un second mélangeur, puis ajouter progressivement les gommes de xanthane, la gomme de cellulose, et la cellulose microcristalline en mélangeant énergiquement jusqu' à homogénéisation. Introduire alors ce mélange (2) dans le mélange (1) en maintenant l'ensemble sous agitation, jusqu'à obtention d'un gel lisse et homogène. Ajouter ensuite le sodium lauroyl sarcosinate, le sodium cocoyl glutamate sous agitation lente, puis le parfum. Enfin, ajuster le pH entre 4.5 et 6.5 avec la solution d'acide citrique. La viscosité de la solution ainsi réalisée se situe dans une fourchette comprise entre1000 et 6000 mPa.s.

**[Table 4]**

| Produits | % (massiques) |
|---|---|
| EAU | 82.099 |
| EXTRAIT DE RIZ | 0.100 |
| PULLULAN | 0.900 |
| SILICE COLLOIDALE | 2.000 |
| ALPHA-GLUCANE OLIGOSACCHARIDE | 0.500 |
| AMIDON DE MAIS | 5.000 |
| GOMME DE CELLULOSE | 0.290 |
| GOMME DE XANTHANE | 0.200 |
| CELLULOSE MICROCRISTALLINE | 0.510 |
| GLYCERINE | 1.500 |
| SODIUM LAUROYL SARCOSINATE | 4.000 |
| SODIUM COCOYL GLUTAMATE | 2.500 |
| PARFUM | 0.400 |
| ACIDE CITRIQUE 5% | 0.001 |
| | 100.00 |

### Etape 2 - Lyophilisation

Verser la solution dans des moules hémisphériques de diamètre 20 mm et congelez ceux-ci à une température de - 40°C pendant deux heures. Assembler les hémisphères deux à deux, mettre à température ambiante pendant 20 minutes pour pouvoir enlever l'un de deux moules hémisphérique. Placez ensuite les hémisphère restant, contenant les billes congelées, dans un lyophilisateur avec une température de chargement de -120°C. Appliquer alors un vide de 10 Pa, puis une température de 30°C, le condenseur étant maintenu à une température de -65°C, ces conditions étant maintenues pendant une durée de 16 heures, à l'issue de laquelle il est possible de récupérer les billes lyophilisées.

### Description de la mise en œuvre pour utilisation.

Placer une bille au creux de la main, et ajouter sur celle-ci quelques millilitres d'eau. Frictionner la bille entre les deux mains l'une contre l'autre jusqu'à ce que la bille se délite, ce qui permet d'obtenir l'action nettoyante recherchée. Rincer après application.

## Revendications

1. Composition cosmétique nettoyante constituée de billes expansées sèches obtenues par la congélation puis la lyophilisation d'une solution aqueuse, **caractérisée en ce que** la solution comporte :
a) Un agent filmogène composé de polysaccharides choisis parmi le pullulan, les alginates, les carraghénanes, les gommes de xanthane ou de guar ou de cellulose, seuls ou en mélange, à une teneur globale comprise entre 0.5 et 10%.
b) Un agent plastifiant choisi parmi les glycols, les polyols, les oligosaccharides, seuls ou en mélange, à une teneur globale comprise entre 0.5 et 10%.
c) Un agent rhéologique constitué d'un mélange comportant d'une part, seuls ou en mélange, de la cellulose microcristalline ou des colloïdes tels que les silices ou les argiles, et d'autre part une poudre d'amidon, à une teneur globale comprise entre 0.1 et 10%.
d) Un mélange de produits tensioactifs dans une proportion comprise entre 0.1 et 10%, préférentiellement choisis parmi les poudres.

2. Composition cosmétique selon la revendication 1, **caractérisée en ce que** le polysaccharide de la solution est composé de pullulan à une teneur comprise entre 0.5 et 5%, associé à de la gomme de xanthane ou de guar ou de cellulose, seuls ou en mélange, dont la teneur globale est comprise entre 0.1 et 5%.

3. Composition cosmétique selon les revendications précédentes, **caractérisée en ce que** l'agent plastifiant de la solution est composé d'un mélange de glycérine à une teneur comprise entre 0.5 et 3% et d'érythritol à une teneur comprise entre 0.5 et 3%.

4. Composition cosmétique selon les revendications précédentes, **caractérisée en ce que** l'agent rhéologique de la solution est composé d'amidon à une teneur comprise entre 2.5 et 7%, associé à de la cellulose microcristalline ou de la bentone à une teneur comprise entre 0.25 et 1.5%.

5. Composition cosmétique selon les revendications précédentes, **caractérisée en ce que** le mélange de produits tensioactifs est constitué de tensioactifs non-ioniques et de tensioactifs anioniques préférentiellement choisis parmi les dérivés de sodium cocoyl ou le disodium lauryl succinate, tels que les sodium methyl cocoyl taurate, sodium cocoyl isethionate, sodium cocoyl glutamate ou du disodium lauryl succinate.

6. Composition cosmétique selon les revendications précédentes, **caractérisée en ce que** le système tensioactif comporte en outre un tensioactif cationique, préférentiellement choisi parmi les ammoniums quaternaires.

7. Procédé de fabrication d'une composition cosmétique selon les revendications précédentes, **caractérisé en ce qu'**il comporte les étapes suivantes :
a) La réalisation de la solution aqueuse.
b) La surgélation ou la congélation de cette solution aqueuse sous forme de billes, soit par un système délivrant la solution en goutte à goutte dans de l'azote liquide, soit en coulant la solution dans des moules sphériques ensuite congelés.
c) La lyophilisation de ces billes.

8. Utilisation d'une composition cosmétique selon les revendications 1 à 6, **caractérisée en ce que** les billes sont placées dans le creux d'une main, puis réhydratées et délitées par ajout de 1 à 5 ml d'eau et friction des mains.

## Patentansprüche

1. Kosmetische Reinigungszusammensetzung, welche aus trockenen expandierten Kügelchen bestehen, die durch Gefrieren und anschließendes Gefriertrocknen einer wässrigen Lösung erhalten werden, **dadurch gekennzeichnet, dass** die Lösung enthält:
a) einen Filmbildner, der aus Polysacchariden besteht, ausgewählt unter Pullulan, Alginaten, Carrageen, Xanthan, Guargummi oder Cellulose, allein oder in einer Mischung, mit einem Gesamtgehalt zwischen 0,5 und 10%;
b) einen Weichmacher, ausgewählt unter Glykolen, Polyolen und Oligosacchariden, allein oder in einer Mischung, mit einem Gesamtgehalt zwischen 0,5 und 10%;
c) ein rheologisches Agens, das aus einer Mischung besteht, die einerseits mikrokristalline Zellulose oder Kolloide wie Kieselerde oder Tonerde allein oder in einer Mischung und andererseits Stärkepulver mit einem Gesamtgehalt von 0,1 bis 10% enthält;
d) eine Mischung von Tensiden in einem Anteil zwischen 0,1 und 10%, vorzugsweise ausgewählt unter Pulvern.

2. Kosmetische Zusammensetzung nach Anspruch 1, **dadurch gekennzeichnet, dass** das Polysaccharid der Lösung aus Pullulan in einem Anteil zwischen 0,5 und 5% besteht, kombiniert mit Xanthan oder Guargummi oder Cellulose, allein oder in einer Mischung, deren Gesamtanteil zwischen 0,1 und 5% liegt.

3. Kosmetische Zusammensetzung nach einem der vorstehenden Ansprüche, **dadurch gekennzeichnet, dass** der Weichmacher der Lösung aus einer Mischung von Glycerin mit einem Gehalt zwischen 0,5 und 3% und Erythrit mit einem Gehalt zwischen 0,5 und 3% besteht.

4. Kosmetische Zusammensetzung nach den vorstehenden Ansprüchen, **dadurch gekennzeichnet, dass** das rheologische Agens der Lösung aus Stärke mit einem Gehalt von 2,5 bis 7% in Kombination mit mikrokristalliner Cellulose oder Bentonit mit einem Gehalt von 0,25 bis 1,5% besteht.

5. Kosmetische Zusammensetzung nach einem der vorstehenden Ansprüche, **dadurch gekennzeichnet, dass** die Mischung von Tensiden aus nichtionischen Tensiden und anionischen Tensiden besteht, vorzugsweise ausgewählt unter Derivaten von Natriumcocoylderivaten oder Dinatriumlaurylsuccinat, wie Natriummethylcocoyltaurat, Natriumcocoylisethionat, Natriumcocoylglutamat oder Dinatriumlaurylsuccinat.

6. Kosmetische Zusammensetzung nach einem der vorstehenden Ansprüche, **dadurch gekennzeichnet, dass** das Tensidsystem zusätzlich ein kationisches Tensid umfasst, vorzugsweise ausgewählt unter quaternären Ammoniumverbindungen.

7. Verfahren zur Herstellung einer kosmetischen Zusammensetzung nach den vorstehenden Ansprüchen, **dadurch gekennzeichnet, dass** es die Schritte umfasst:
a) Herstellung der wässrigen Lösung;
b) Tiefgefrieren oder Gefrieren der wässrigen Lösung in die Form von Kügelchen, entweder durch ein System, das die Lösung tropfenweise in flüssigem Stickstoff abgibt, oder durch Gießen der Lösung in kugelförmige Formen, die dann gefroren werden;
c) Gefriertrocknen der Kügelchen.

8. Verwendung einer kosmetischen Zusammensetzung nach einem der Ansprüche 1 bis 6, **dadurch gekennzeichnet, dass** man die Kügelchen in eine Handfläche gibt, dann rehydriert und auflöst, indem man 1 bis 5 ml Wasser zugibt und die Hände aneinander reibt.

## Claims

1. Cosmetic cleansing composition consisting of dry expanded beads obtained by freezing and then freeze-drying an aqueous solution, **characterized in that** the solution comprises :
a) A film-forming agent composed of polysaccharides chosen from pullulan, alginates, carrageenans, xanthan or guar gums or cellulose, alone or as a mixture, in a total content of between 0.5 and 10%.
b) A plasticising agent chosen from glycols, polyols and oligosaccharides, alone or as a mixture, at a total content of between 0.5 and 10%.
c) A rheological agent consisting of a mixture comprising on the one hand, alone or as a mixture, microcrystalline cellulose or colloids such as silicas or clays, and on the other hand starch powder, in a total content of between 0.1 and 10%.
d) A mixture of surfactants in a proportion of between 0.1 and 10%, preferably chosen from powders.

2. Cosmetic composition according to claim 1, **characterized in that** the polysaccharide of the solution is composed of pullulan at a content of between 0.5 and 5%, combined with xanthan gum or guar gum or cellulose, alone or as a mixture, the overall content of which is between 0.1 and 5%.

3. Cosmetic composition according to the preceding claims, **characterized in that** the plasticizing agent of the solution is composed of a mixture of glycerine at a content of between 0.5 and 3% and erythritol at a content of between 0.5 and 3%.

4. Cosmetic composition according to the preceding claims, **characterized in that** the rheological agent of the solution is composed of starch at a content of between 2.5 and 7%, combined with microcrystalline cellulose or bentone at a content of between 0.25 and 1.5%.

5. Cosmetic composition according to the preceding claims, **characterized in that** the mixture of surfactant products consists of non-ionic surfactants and anionic surfactants preferably chosen from sodium cocoyl derivatives or disodium lauryl succinate, such as sodium methyl cocoyl taurate, sodium cocoyl isethionate, sodium cocoyl glutamate or disodium lauryl succinate.

6. Cosmetic composition according to the preceding claims, **characterized in that** the surfactant system additionally comprises a cationic surfactant, preferably chosen from quaternary ammoniums.

7. Method of manufacturing a cosmetic composition according to the preceding claims, **characterized in that** it comprises the following steps
a) Making the aqueous solution.
b) This aqueous solution is deep-frozen or frozen in the form of beads, either by a system delivering the solution drop by drop in liquid nitrogen, or by pouring the solution into spherical moulds which are then frozen
c) Freeze-drying these beads.

8. Use of a cosmetic composition according to claims 1 to 6, **characterized in that** the beads are placed in the palm of a hand, then rehydrated and disintegrated by adding 1 to 5 ml of water and rubbing the hands together.
